# FASCICULE DE BREVET EUROPEEN

(11) **EP 4 264 104 B1**
(45) Date de publication et mention de la délivrance du brevet: **11.09.2024**
(21) Numéro de dépôt: 21839983.0
(22) Date de dépôt: 16.12.2021
(51) Int. Cl.: F16L 25/00, A61M 39/18, B29C 65/00, F16L 29/00, F16L 47/00, A61M 39/26

(54) **DISPOSITIF DE RACCORDEMENT ASEPTIQUE D'UN TUBE**
ASEPTISCHE KUPPLUNGSVORRICHTUNG FÜR EIN ROHR
ASEPTIC COUPLING DEVICE FOR A TUBE

(30) Priorité: 16.12.2020 FR 2013403
(43) Date de publication de la demande: 25.10.2023
(73) Titulaire: Parker Hannifin EMEA S.à.r.l., 1163 Etoy (CH)
(72) Inventeur: LE QUERE, Philippe, 35830 Betton (FR)
(74) Mandataire: Cabinet Boettcher
(86) Numéro de dépôt international: PCT/EP2021/086312
(87) Numéro de publication internationale: WO 2022/129413

(56) Documents cités:
- WO-A1-2013/147688
- WO-A1-2017/102482
- WO-A1-98/50105
- FR-A1- 2 953 903

## Description

La présente invention concerne le domaine du transport de fluides liquides ou gazeux et plus particulièrement un dispositif de raccordement aseptique destiné à réaliser le raccordement de deux tubes stériles.

### ARRIERE PLAN DE L'INVENTION

On connaît des ensembles de raccordement destinés à connecter des canalisations stériles reliées à des éléments de circuits de l'industrie pharmaceutiques, tels que des filtres, des centrifugeuses, des pompes, des réservoirs en plastique souple ou des conteneurs de traitement comme des bio-processeurs.

Un tel ensemble comprend généralement deux dispositifs de raccordement destinés à être connectés entre eux après que chacun ait été raccordé à une extrémité de canalisation. Chaque dispositif comprend un corps tubulaire délimitant un canal agencé pour recevoir l'extrémité de canalisation. Le corps comporte une face frontale sur laquelle est fermement fixé un élément d'étanchéité annulaire avec lequel l'extrémité de canalisation est destinée à rentrer en contact étanche. L'élément d'étanchéité est recouvert d'un film de protection en forme de de bande repliée sur elle-même dont une partie adhère de manière amovible à un flanc latéral libre de l'élément d'étanchéité et une autre partie forme une languette de tirage s'étendant en saillie de la première partie.

Après que les extrémités de canalisation aient été emmanchées dans les corps, les dispositifs sont mis coaxialement en regard puis rapprochés l'un de l'autre jusqu'à mettre en contact les films de protection et comprimer légèrement les éléments d'étanchéité. Le retrait simultané des films de protection par tirage des languettes entraîne une mise en contact étanche des flancs latéraux des éléments d'étanchéité entre eux et donc la formation d'un canal aseptique de passage de fluide entre les deux canalisations. Un rapprochement supplémentaire des dispositifs est réalisé avant de verrouiller les positions relatives des dispositifs au moyen d'une bride de serrage.

A la fin d'un processus de fabrication d'un lot de produits pharmaceutiques, l'ensemble des canalisations et des dispositifs de raccordement sont généralement démontés et incinérés. Leur reconditionnement (lavage, stérilisation...) s'avère en effet coûteux et présente un risque de dégradation des dispositifs (usure, rupture ou bien encore perte d'un composant).

Néanmoins, il apparaît que de tels dispositifs comportent de nombreux composants les rendant particulièrement coûteux pour un usage unique, et cela d'autant plus lorsque celui-ci est de courte durée.

Il serait par ailleurs souhaitable de limiter voire supprimer l'écoulement du fluide resté prisonnier dans les canalisations lors de la déconnexion des dispositifs. Pour cela, il a été envisagé d'équiper les dispositifs de raccordement de valves. On connaît en effet des dispositifs de raccordement lavables et réutilisables comprenant des valves prévues à cet effet. Il ne serait cependant pas viable économiquement d'équiper les dispositifs de raccordement jetables de telles valves.

Le document WO 98/50105 A divulgue un dispositif de raccordement aseptique d'un tube qui comprend un insert tubulaire pourvu de moyens de sa connexion à un tube et une portion destinée à s'étendre à l'extérieur du tube, un élément d'étanchéité disposé en face frontale de l'insert, l'élément d'étanchéité comprenant une face frontale en saillie sur laquelle est fixé de manière amovible un film de protection.ce document présente également un élément vanne monté hors de l'insert.

### OBJET DE L'INVENTION

L'invention a donc pour but de proposer un dispositif de raccordement aseptique d'un tube permettant d'obvier au moins en partie aux problèmes précités.

### RESUME DE L'INVENTION

A cet effet, l'invention propose un dispositif de raccordement aseptique d'un tube comprenant :
- un insert tubulaire délimitant un canal et ayant un premier tronçon d'extrémité pourvu de moyens de sa connexion au tube et un deuxième tronçon d'extrémité qui est destiné à s'étendre à l'extérieur du tube, l'insert tubulaire étant pourvu d'une tige s'étendant axialement en porte-à-faux dans le canal pour avoir une portion d'extrémité libre en saillie du deuxième tronçon d'extrémité de l'insert ; et
- un corps annulaire comprenant un premier tronçon d'extrémité monté sur le deuxième tronçon d'extrémité de l'insert et un deuxième tronçon d'extrémité s'étendant axialement en saillie du deuxième tronçon d'extrémité de l'insert pour définir avec celui-ci, en regard de la portion d'extrémité libre de la tige, un logement de réception d'un élément d'étanchéité annulaire du dispositif qui est disposé entre une face frontale du deuxième tronçon d'extrémité de l'insert et un redan interne du deuxième tronçon du corps annulaire.
Selon l'invention :
- élément d'étanchéité annulaire est déformable depuis un premier état dans lequel l'élément d'étanchéité délimite une section de passage supérieure à une section transversale de la tige et un deuxième état dans lequel l'élément d'étanchéité enserre la tige, l'élément d'étanchéité étant agencé pour être amené de son premier état vers son deuxième état sous l'effet d'une compression axiale.
- l'élément d'étanchéité comprend une face frontale en saillie du deuxième tronçon du corps annulaire et sur laquelle est fixé de manière amovible un film de protection sur lequel est rabattue une languette de tirage ayant une première extrémité reliée à un bord du film de protection et une deuxième extrémité s'étendant en saillie d'un bord du film opposé à la première extrémité de la languette de tirage ; et
- le premier tronçon d'extrémité du corps est monté sur l'insert pour être déplaçable axialement entre une position en saillie axiale de l'insert dans laquelle l'élément d'étanchéité est dans son premier état et une position affleurant l'insert dans laquelle l'élément d'étanchéité est suffisamment comprimé axialement pour être dans son deuxième état.

Ainsi, l'élément d'étanchéité permet à la fois d'assurer l'étanchéité du raccordement du tube et d'obstruer totalement le canal, et donc de limiter le nombre de composant du dispositif tout en empêchant l'écoulement du fluide resté prisonnier dans le tube lors de la déconnexion du dispositif.

Selon une caractéristique particulière de l'invention, le dispositif comprend des moyens de verrouillage du corps en position en saillie et en position affleurante.

Selon une autre caractéristique particulière de l'invention, le dispositif comprend des moyens de pré-couplage et de couplage à un autre dispositif de raccordement.

De manière particulière, les moyens de pré-couplage et de couplage comprennent au moins une patte de verrouillage s'étendant en saillie d'une face frontale du corps et comprenant à une extrémité libre au moins deux aspérités destinées à coopérer avec un bord de l'autre dispositif de raccordement.

L'invention concerne également un ensemble de raccordement de deux tubes comprenant deux tels dispositifs de raccordement.

Avantageusement, les deux dispositifs de raccordement sont identiques.

L'invention concerne aussi un procédé de raccordement de deux tubes à l'aide d'un tel ensemble de raccordement, le procédé comprenant les étapes suivantes :
- disposer coaxialement les dispositifs en regard l'un de l'autre ;
- rapprocher les dispositifs l'un de l'autre jusqu'à ce qu'à ce les languettes de tirage soient en contact l'une avec l'autre et les films de protections plaquées contre la face frontale des corps ;
- retirer les films de protection en tirant simultanément sur les languettes de tirage ;
- rapprocher de nouveau les dispositifs l'un de l'autre jusqu'à ce que les corps soient plaqués l'un contre l'autre.

### BREVE DESCRIPTION DES DESSINS

L'invention sera mieux comprise à la lumière de la description qui suit, laquelle est purement illustrative et non limitative, et doit être lue en regard des dessins annexés, parmi lesquels :
- la figure 1 est une vue en coupe axiale d'un dispositif de raccordement selon un mode de réalisation particulier de l'invention ;
- la figure 2 est une vue en coupe transversale du dispositif de raccordement illustré à la figure 1 ;
- la figure 3 est une vue de face du dispositif de raccordement illustré à la figure 1 ;
- la figure 4A est une vue en coupe axiale d'un ensemble de raccordement aseptique selon un mode de réalisation particulier de l'invention, avant connexion ;
- la figure 4B est une vue analogue à la figure 4A pendant la connexion, avant le retrait du film de protection ;
- la figure 4C est une vue analogue à la figure 4A pendant la connexion, après le retrait du film de protection ;
- la figure 4D est une vue analogue à la figure 4A après connexion ;
- la figure 5A une vue analogue à la figure 4D, avant déconnexion ;
- la figure 5B une vue analogue à la figure 5B pendant la déconnexion.
- la figure 5C une vue analogue à la figure 5A après déconnexion.

### DESCRIPTION DETAILLEE DE L'INVENTION

L'invention est ici décrite en application au raccordement d'un premier tube 100 stérile formant un premier conduit de transport de fluide à un deuxième conduit de transport de fluide formé par un deuxième tube 200 stérile.

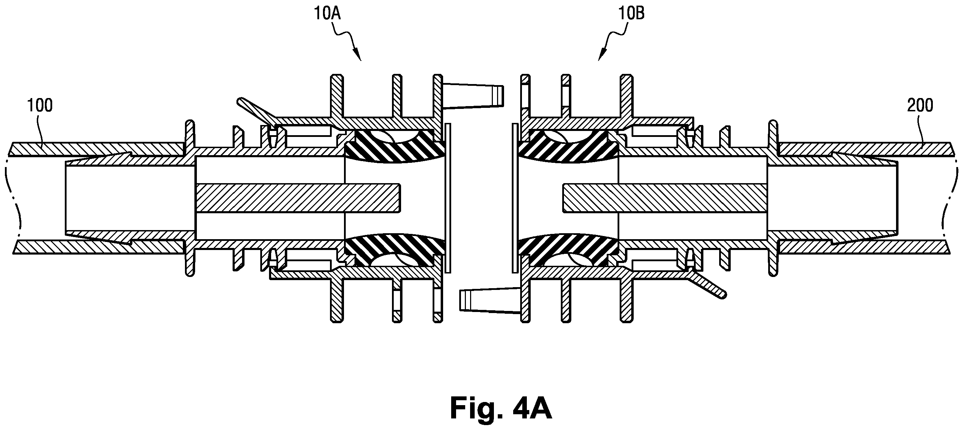

En référence aux figures 4A à 4D, un ensemble de raccordement fluidique généralement désigné en 1, selon un mode de réalisation particulier de l'invention, comprend deux dispositifs 10 de raccordement destinés à réaliser ensemble une connexion aseptique entre le premier tube 100 et le deuxième tube 200. Les deux dispositifs 10 sont identiques et sont distingués sur les figures par l'adjonction des lettres A, B.

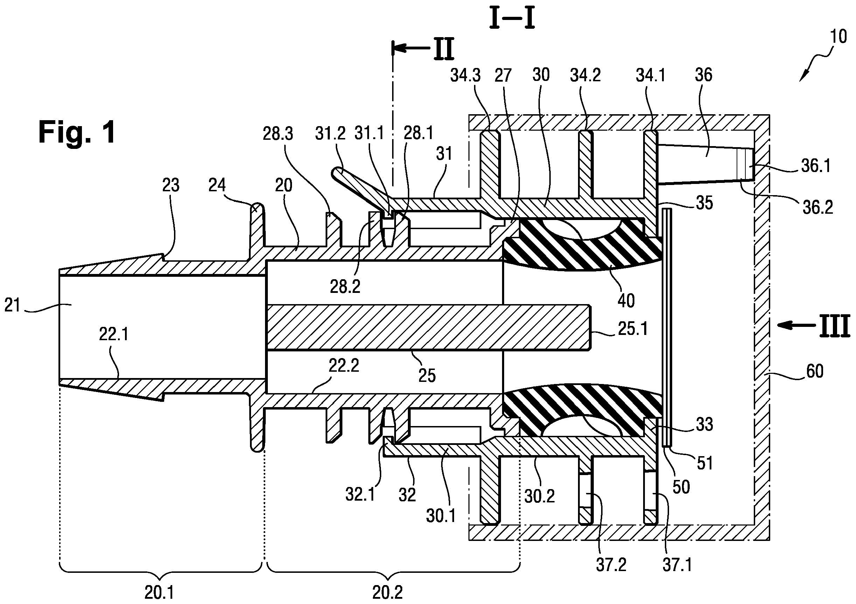

Comme illustré à la figure 1, chaque dispositif 10 comprend un embout ou insert 20 tubulaire délimitant un canal 21 de passage de fluide.

L'insert 20 comporte un premier tronçon 20.1 d'extrémité délimitant un premier alésage 22.1 formant une portion du canal 21. Le premier tronçon 20.1 comprend extérieurement un relief 23 en dent de sapin et une collerette 24 formant respectivement un moyen de son ancrage dans le tube 100, 200 et une butée à son enfoncement dans ledit tube 100, 200.

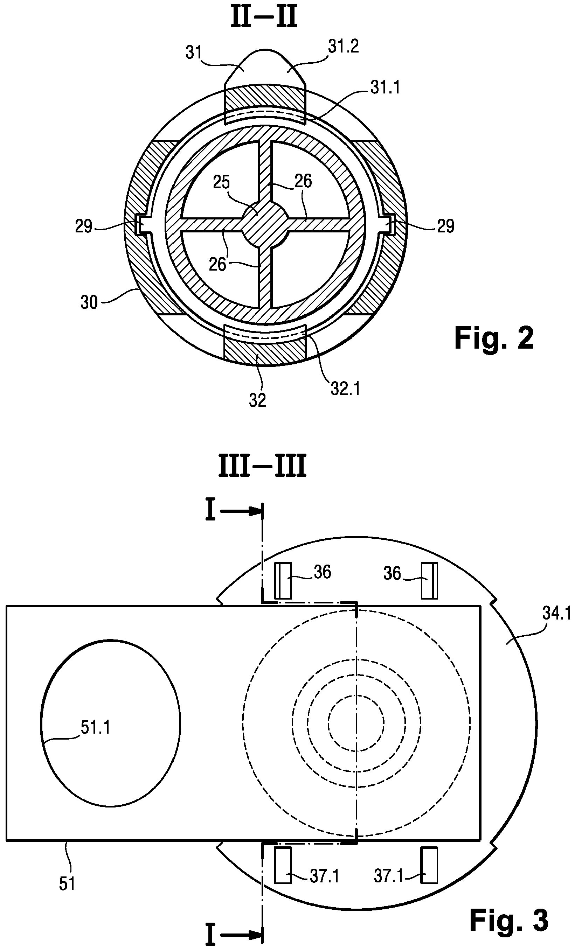

L'insert 20 comporte un deuxième tronçon 20.2 d'extrémité délimitant un deuxième alésage 22.2 coaxial au premier alésage 22.1 et formant une portion du canal 21. Le deuxième tronçon 20.2 est pourvu intérieurement d'une tige 25 de forme cylindrique s'étendant axialement en porte à faux dans le canal 21 de sorte qu'une portion d'extrémité libre 25.1 de la tige s'étend en saillie du deuxième tronçon 20.2 de l'insert 20. Comme illustré à la figure 2, la tige 25 est reliée au deuxième alésage 22.2 via quatre ailettes 26 équitablement réparties autour de ladite tige 25. Le diamètre de la tige 25 est ici tel que la section de passage de fluide du canal 21 est sensiblement identique dans le premier tronçon 20.1 et le deuxième tronçon 20.2.

Le deuxième tronçon 20.2 de l'insert 20 est également pourvu extérieurement d'une collerette 27 agencée à une extrémité libre dudit deuxième tronçon 20.2 et de trois anneaux de crantage 28.1, 28.2, 28.3 coaxiaux ayant des diamètres extérieurs sensiblement identiques. La collerette 27 définit une face frontale du deuxième tronçon 20.2 de l'insert 20 et forme un premier moyen de centrage d'un corps 30 tubulaire monté sur l'insert 20. Les anneaux de crantage 28.1, 28.2, 28.3 forment des deuxièmes moyens de centrage du corps 30 sur l'insert 20 et des moyens de verrouillage en position du corps 30 sur l'insert 20 comme on le verra plus loin.

Le corps 30 comprend un premier tronçon 30.1 d'extrémité monté sur le deuxième tronçon 20.2 de l'insert 20, et un deuxième tronçon 30.2 d'extrémité s'étendant axialement en saillie dudit deuxième tronçon 20.2 de l'insert 20.

Le premier tronçon 30.1 comporte quatre rainures axiales débouchantes sur un bord libre du premier tronçon 30.1 pour délimiter entre elles deux lames 31, 32 diamétralement opposées ayant une extrémité solidaire du reste du premier tronçon 30.1 et une extrémité opposée comprenant un relief 31.1, 32.1 interne coopérant avec un flanc latéral du premier anneau de crantage 28.1. Les lames 31, 32 sont élastiquement déformables entre un état de repos dans laquelle les reliefs 31.1, 32.1 délimitent une section de passage inférieure au diamètre des anneaux de crantages 28.1, 28.2, 28.3 de l'insert 20 et un état déformé dans lequel lesdits reliefs 31.1, 32.1 délimitent une section de passage supérieure au diamètre desdits anneaux de crantages 28.1, 28.2, 28.3. La lame 31 diffère de la lame 32 en ce qu'elle comprend à une extrémité libre une extension 31.2 agencée pour recevoir un effort de soulèvement permettant d'amener la lame 31 à l'état déformé.

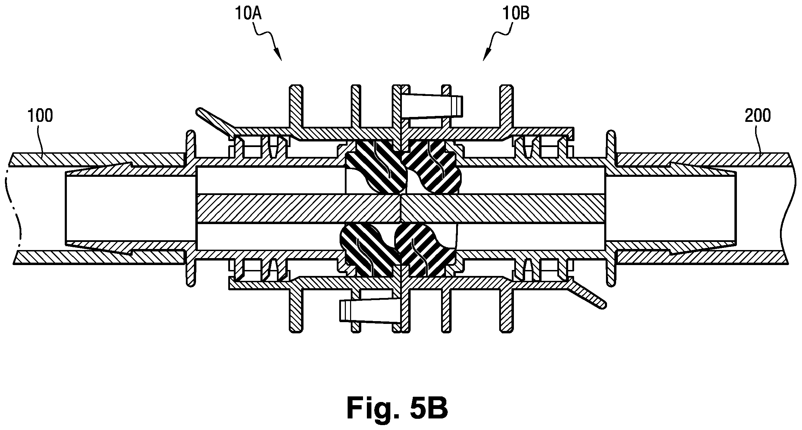

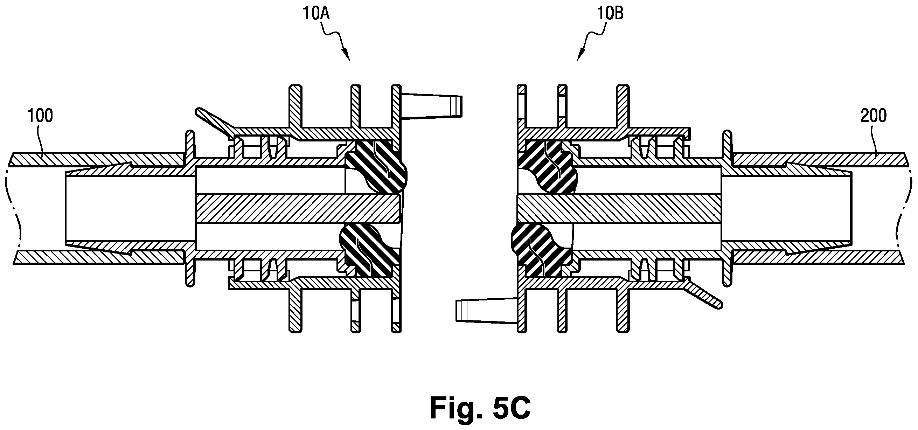

Les anneaux de crantage 28.1, 28.2, 28.3 de l'insert 20 ont un pourtour extérieur comportant un chanfrein agencé pour ne pas globalement s'opposer au montage du corps 30 sur l'insert 20 ni à un déplacement axial du corps 30 depuis une position en saillie dans laquelle les reliefs 31.1, 32.1 des lames 31, 32 coopèrent avec le flanc latéral du premier anneau de crantage 28.1 (figures 1, 4A-4D) vers une position affleurant l'insert 20 dans laquelle lesdits reliefs 31.1, 32.1 coopèrent avec un flanc latéral du troisième anneau de crantage 28.3 (figure 5B et 5C), mais aussi pour s'opposer à un déplacement axial du corps 30 depuis la position affleurante vers la position en saillie. Comme cela est visible sur la figure 1, seul le pourtour du deuxième anneau de crantage 28.2 est localement non chanfreiné pour empêcher un déplacement axial du corps 30 depuis la position en saillie vers la position affleurante sans application d'un effort de soulèvement suffisant sur la lame 31 pour l'amener à l'état déformé.

Conformément à la figure 2, le pourtour des anneaux de crantage 28.1, 28.2, 28.3 comportent extérieurement deux excroissances 29 diamétralement opposées et agencées pour être reçues dans deux gorges aménagées dans le corps 30 de manière à lier en rotation le corps 30 à l'insert 20.

Le deuxième tronçon 30.2 du corps 30 définit avec le deuxième tronçon 20.2 de l'insert 20, en regard de la portion d'extrémité 25.1 de la tige 25, un logement de réception d'un élément d'étanchéité 40 annulaire qui est disposé entre la face frontale du deuxième tronçon 20.2 formée par la collerette 27 et un redan 33 annulaire interne du deuxième tronçon 30.2 agencé à une extrémité libre dudit deuxième tronçon 30.2.

Le deuxième tronçon 30.2 du corps 30 est pourvu extérieurement de trois couronnes 34.1, 34.2, 34.3 formant des moyens de préhension du dispositif 10. Les couronnes 34.1, 34.2, 34.3 sont coaxiales et ont des diamètres extérieurs sensiblement identiques. La première couronne de préhension 34.1 s'étend radialement dans le prolongement du redan 33 et définit avec celui-ci une face frontale 35 du corps 30 destinée à coopérer avec la face frontale d'un corps d'un autre dispositif 10.

On notera que lorsque le corps 30 est en position affleurante, la face frontale 35 dudit corps 30 et l'extrémité libre de la portion d'extrémité 25.1 de la tige 25 s'étendent ici sensiblement dans un même plan.

Deux pattes de verrouillage 36, élastiquement déformables et ici de forme sensiblement parallélépipédique, s'étendent axialement en saillie de la face frontale 35 du corps 30, au voisinage d'un pourtour de celle-ci. Comme illustré à la figure 3, les pattes de verrouillage 36 sont symétriques et s'étendent parallèlement au plan de la figure 1. Une extrémité libre des pattes de verrouillage 36 a une surface extérieure pourvue de deux aspérités 36.1, 36.2 en dent de sapin destinée à assurer un pré-accouplement et un accouplement du dispositif 10 avec l'autre dispositif 10.

La première couronne 34.1 et la deuxième couronne 34.2 du corps 30 sont respectivement pourvues de deux premières fentes 37.1 et de deux deuxièmes fentes 37.2 de forme sensiblement rectangulaire destinées à recevoir chacune une portion des pattes de verrouillage 36 de l'autre dispositif 10. Les fentes 37.1 de la première couronne 34.1 sont agencées au voisinage du pourtour de la collerette 37 et sont diamétralement opposées aux pattes de verrouillage 36. Les fentes 37.2 de la deuxième couronne 34.2 sont en regard des fentes 37.1 de la première couronne 34.1 et ont un bord destiné à coopérer avec les aspérités 36.1, 36.2 des pattes de verrouillage 36 de l'autre dispositif 10.

L'élément d'étanchéité 40 est en contact étanche avec la face frontale du deuxième tronçon 20.2 et un flanc interne du redan 33 en regard de ladite face frontale. L'élément d'étanchéité 40 est déformable depuis un premier état dans lequel il délimite une section de passage supérieure à une section transversale de la tige 25 de l'insert 20 et un deuxième état dans lequel il enserre la tige 25, et est agencé pour être amené de son premier état vers son deuxième état sous l'effet d'une compression axiale. Un tel élément d'étanchéité est connu du document EP2569665.

Lorsque le corps 30 est déplacé axialement de la position en saillie à la position affleurante, l'insert 20 comprime suffisamment l'élément d'étanchéité 40 contre le redan 33 pour l'amener de son premier état à son deuxième état.

L'élément d'étanchéité 40 comprend une face frontale s'étendant en saillie du deuxième tronçon 30.2 du corps 30 et sur laquelle est fixé de manière amovible un film de protection 50 sur laquelle est rabattue une languette 51 de tirage permettant de retirer le film de protection 50 sans toucher à celui-ci. La languette 51 de tirage a une première extrémité reliée à un bord du film de protection 50 et une deuxième extrémité s'étendant en saillie d'un bord du film de protection 50 opposé à la première extrémité de la languette 51. La deuxième extrémité de la languette 51 comprend en son centre un trou 51.1 conformé pour permettre le passage d'un doigt à travers ledit trou 51.1 de manière à améliorer la préhension de la languette 51.

Afin d'éviter toute détérioration et contamination de l'élément d'étanchéité 40 lors du stockage et du transport du dispositif 10, un capot 60 de protection est monté de manière amovible sur le deuxième tronçon 30.2 du corps 30. Le capot 60 peut par exemple être agencée pour se clipper (par emboîtement élastique) sur la troisième couronne 34.3 de préhension du corps 30 et est destiné à être retiré juste avant le couplage du dispositif 10 à l'autre dispositif 10.

On notera que l'ensemble du dispositif 10 est stérilisé en usine afin d'être exempt de toute contamination avant son stockage.

Le fonctionnement de l'ensemble de raccordement 1 va maintenant être détaillé en regard des figures 4A à 5C.

Le tronçon 20.1 d'extrémité du premier dispositif 10A est tout d'abord emmanché dans le premier tube 100 jusqu'à ce que celui-ci vienne en butée contre la collerette 24 dudit premier dispositif 10A. De même, le tronçon 20.1 d'extrémité du deuxième dispositif 10B est emmanché dans le deuxième tube 200 jusqu'à ce que celui-ci vienne en butée contre la collerette 24 dudit deuxième dispositif 10B, de sorte que les premier et deuxième dispositifs 10A, 10B sont respectivement ancrés dans les premier et deuxième tubes 100, 200.

Les capots 60 de protection des premier et deuxième dispositifs 10A, 10B sont alors retirés des corps 30 rendant notamment visibles les pattes de verrouillage 36 et les fentes 37.1, 37.2 desdits corps 30.

Les premier et deuxième dispositifs 10A, 10B sont ensuite présentés coaxialement en regard l'un de l'autre, le premier dispositif 10A étant tourné axialement de 180 degrés par rapport au deuxième dispositif 10B de sorte que les pattes de verrouillage 36 et les fentes 37.1, 37.2 du premier dispositif 10A sont respectivement en regard des fentes 37.1, 37.2 et des pattes de verrouillage 36 du deuxième dispositif 10B (figure 4A).

Dans un premier temps, les premier et deuxième dispositifs 10A, 10B sont rapprochés l'un de l'autre jusqu'à ce que les languettes 51 de tirage des premier et deuxième dispositifs 10A, 10B soient en contact, les fentes 37.1, 37.2 du premier dispositif 10A recevant alors chacune une portion des pattes de verrouillage 36 du deuxième dispositif 10B et les fentes 37.1, 37.2 du deuxième dispositif 10B recevant chacune une portion des pattes de verrouillage 36 du premier dispositif 10A.

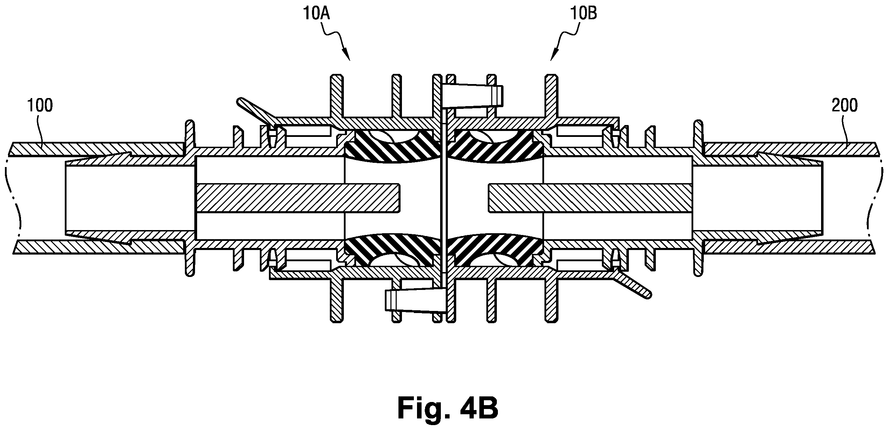

En poursuivant le rapprochement des premier et deuxième dispositifs 10A, 10B, les films de protection 50 sont plaqués contre la face frontale 35 des corps 30 formé par le redan 33 et la première couronne 34.1, ce qui entraîne une légère compression d'une portion d'extrémité des éléments d'étanchéité 40 s'étendant en saillie de ladite face frontale mais aussi une coopération des premières aspérités 36.1 des pattes de verrouillage 36 du premier dispositif 10A avec le bord des deuxièmes fentes 37.2 du deuxième dispositif 10B et une coopération des premières aspérités 36.1 des pattes de verrouillage 36 du deuxième dispositif 10B avec le bord des deuxièmes fentes 37.2 du premier dispositif 10A (figure 4B). On parle alors de pré-couplage des premier et deuxième dispositifs 10A, 10B.

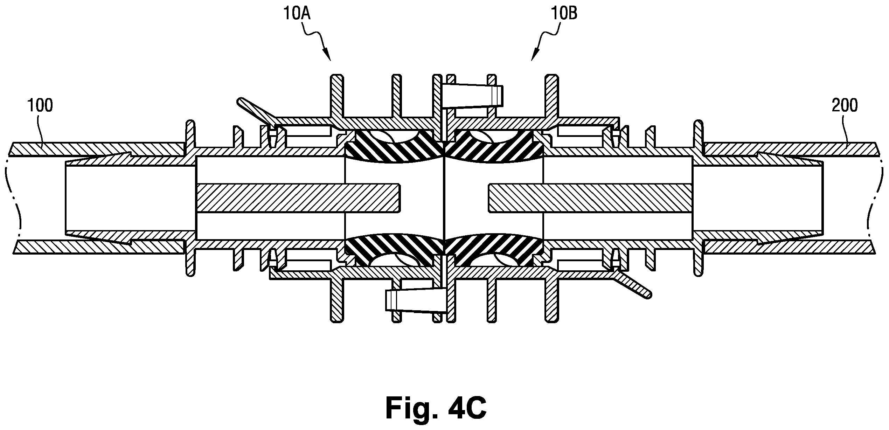

Les films de protection 50 sont alors retirés en passant un même doigt à travers les trous 51.1 des languettes 51 des premier et deuxième dispositifs 10A, 10B et en tirant simultanément sur lesdites languettes 51, provoquant alors une décompression des portions d'extrémité des éléments d'étanchéité et une mise en contact étanche de celles-ci (figure 4C).

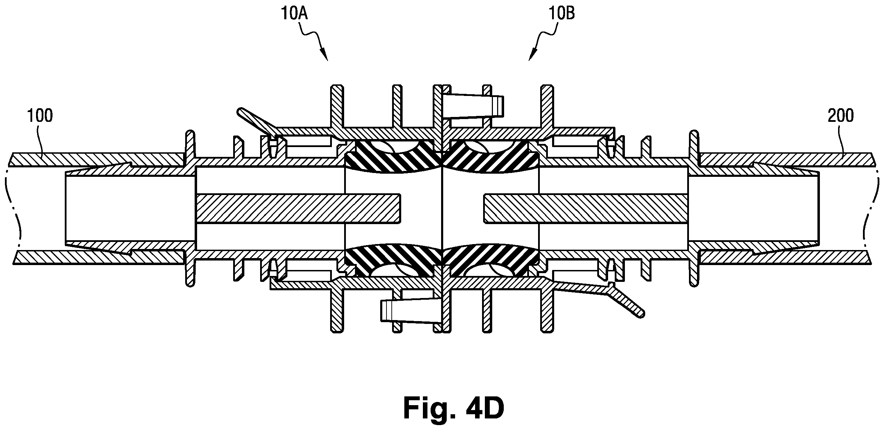

Un dernier rapprochement des premier et deuxième dispositifs 10A, 10B est ensuite réalisé pour plaquer les faces frontales 35 des corps 30 l'une contre l'autre, provoquant alors de nouveau une légère compression des portions d'extrémité des éléments d'étanchéité 40 et une coopération des deuxièmes aspérités 36.2 des pattes de verrouillage 36 avec les bords des deuxièmes fentes 37.2 (figure 4D). On parle alors de couplage des premier et deuxième dispositifs 10A, 10B qui forment ainsi ensemble un canal pour le passage d'un fluide entre le premier tube 100 et le deuxième tube 200.

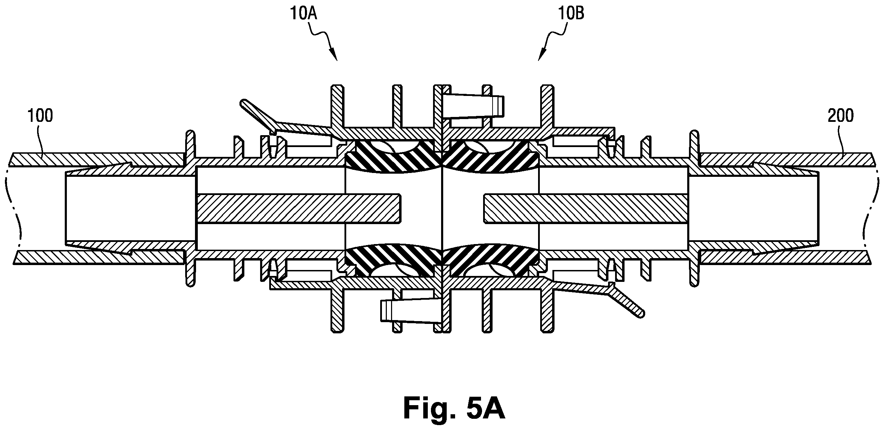

Lorsqu'il convient de déconnecter le premier dispositif 10A du deuxième dispositif 10B, un effort de soulèvement est tout d'abord exercé sur les extensions 31.2 des premières lames 31 du corps 30 de manière à amener lesdites premières lames 15 à l'état déformé et ainsi permettre aux corps 30 de se déplacer axialement vers la position affleurante (figure 5A).

Un effort axial est ensuite exercé sur les inserts 20, par exemple via les tubes 100, 200, jusqu'à amener les corps 30 en position affleurante et ainsi comprimer suffisamment les éléments d'étanchéité 40 pour qu'ils enserrent les tiges 25 des inserts 20 (figure 5B). Les extrémités libres des portions d'extrémité 25.1 des tiges 25 sont alors sensiblement en contact et le fluide ne peut plus circuler entre le premier tube 100 et le deuxième tube 200, et plus particulièrement entre le premier dispositif 10A et le deuxième dispositif 10B.

Dès lors que les reliefs 31.1 des premières lames 31 ont dépassé les deuxièmes anneaux de crantage 28.2, l'effort de soulèvement exercé sur lesdites premières lames 31 est stoppé, les reliefs 31.1 ne s'opposant alors plus au déplacement des corps 30 vers la position affleurante.

Une fois en position affleurante, les reliefs 31.1, 32.1 des lames 31, 32 s'opposent au déplacement axial des corps 30 vers la position en saillie sous l'effet des efforts engendrés par la compression des éléments d'étanchéités 40. Un effort de pincement est alors simultanément exercé sur les pattes de verrouillage 36 dudit premier dispositif 10A et sur les pattes de verrouillage 36 du deuxième dispositif 10B de manière à déformer élastiquement lesdites pattes de verrouillage 36 et permettre aux corps 30 d'être désolidariser l'un de l'autre (figure 5C). L'enserrement des tiges 25 par les éléments d'étanchéité 40 permet ainsi d'empêcher l'écoulement du fluide resté prisonnier dans les tubes 100, 200 lors de la déconnexion des dispositifs 10A, 10B.

Bien entendu, l'invention n'est pas limitée au mode de réalisation décrit mais englobe toute variante entrant dans le champ de l'invention telle que définie par les revendications.

Bien que l'ensemble de raccordement comprenne ici deux dispositifs 10A, 10B identiques, il peut aussi comprendre deux dispositifs différents. Par exemple, les pattes de verrouillage 36 du premier dispositif 10A peuvent être remplacées par des fentes et les fentes 37.1, 37.2 du deuxième dispositif 10B peuvent être remplacées par des pattes de verrouillage de manière à former un connecteur mâle et un connecteur femelle.

Bien que l'accouplement des dispositifs 10A, 10B soit ici réalisé par clippage, il peut aussi être réalisé par tout autre moyen tel que par vissage d'une partie du dispositif 10A sur l'autre dispositif 10B.

Bien que l'ancrage des tubes 100, 200 sur les dispositifs 10A, 10B soit ici réalisé par un relief en dent de sapin, il peut aussi être réalisé par tout autre moyen tel qu'un jonc, une rondelle extérieurement dentée ou des bras élastiquement déformables.

Bien que le verrouillage du corps 30 en position en saillie et en position soit ici réalisé par l'utilisation d'anneaux de crantage, il peut aussi être réalisé par tout autre moyen tel que par l'utilisation de piges.

Bien que l'élément d'étanchéité soit ici symétrique, il peut être non symétrique.

Les étapes nécessaires à la déconnexion des dispositifs 10A, 10B peuvent être réalisées simultanément sur chacun des dispositifs 10A, 10B ou bien sur l'un des dispositifs puis l'autre.

## Revendications

1. Dispositif (10) de raccordement aseptique d'un tube, le dispositif comprenant
- un insert (20) tubulaire délimitant un canal (21) et ayant un premier tronçon (20.1) d'extrémité pourvu de moyens de sa connexion au tube et un deuxième tronçon (20.2) d'extrémité qui est destiné à s'étendre à l'extérieur du tube, l'insert tubulaire étant pourvu d'une tige (25) s'étendant axialement en porte-à-faux dans le canal pour avoir une portion d'extrémité (25.1) libre en saillie du deuxième tronçon d'extrémité de l'insert ; et
- un corps (30) annulaire comprenant un premier tronçon (30.1) d'extrémité monté sur le deuxième tronçon d'extrémité de l'insert et un deuxième tronçon (30.2) d'extrémité s'étendant axialement en saillie du deuxième tronçon d'extrémité de l'insert pour définir avec celui-ci, en regard de la portion d'extrémité libre de la tige, un logement de réception d'un élément d'étanchéité (40) annulaire du dispositif qui est disposé entre une face frontale du deuxième tronçon d'extrémité de l'insert et un redan (33) interne du deuxième tronçon du corps annulaire et qui est déformable depuis un premier état dans lequel l'élément d'étanchéité délimite une section de passage supérieure à une section transversale de la tige et un deuxième état dans lequel l'élément d'étanchéité enserre la tige, l'élément d'étanchéité étant agencé pour être amené de son premier état vers son deuxième état sous l'effet d'une compression axiale ;
et dans leque1:
- l'élément d'étanchéité comprend une face frontale en saillie du deuxième tronçon du corps annulaire et sur laquelle est fixé de manière amovible un film de protection (50) sur lequel est rabattue une languette (51) de tirage ayant une première extrémité reliée à un bord du film de protection et une deuxième extrémité s'étendant en saillie d'un bord du film opposé à la première extrémité de la languette de tirage ; et
- le premier tronçon d'extrémité du corps est monté sur l'insert pour être déplaçable axialement entre une position en saillie axiale de l'insert dans laquelle l'élément d'étanchéité est dans son premier état et une position affleurant l'insert dans laquelle l'élément d'étanchéité est suffisamment comprimé axialement pour être dans son deuxième état.

2. Dispositif selon la revendication 1, comprenant des moyens de verrouillage (28.1, 28.2, 28.3, 31, 32) du corps (30) en position en saillie et en position affleurante.

3. Dispositif selon la revendication 1, comprenant des moyens de pré-couplage et de couplage (36, 37.1, 37.2) à un autre dispositif de raccordement.

4. Dispositif selon la revendication 3, dans lequel les moyens de pré-couplage et de couplage comprennent au moins une patte de verrouillage s'étendant en saillie d'une face frontale du corps (30) et comprenant à une extrémité libre au moins deux aspérités (36.1, 36.2) destinées à coopérer avec un bord de l'autre dispositif de raccordement.

5. Ensemble (1) de raccordement de deux tubes (100, 200) comprenant deux dispositifs (10A, 10B) de raccordement selon l'une quelconque des revendications précédentes.

6. Ensemble (1) selon la revendication 5, dans lequel les deux dispositifs (10A, 10B) de raccordement sont identiques.

7. Procédé de raccordement de deux tubes (100, 200) à l'aide d'un ensemble de raccordement selon les revendications 5 ou 6, le procédé comprenant les étapes suivantes :
- disposer coaxialement les dispositifs en regard l'un de l'autre ;
- rapprocher les dispositifs l'un de l'autre jusqu'à ce qu'à ce les languettes (51) de tirage soient en contact l'une avec l'autre et les films de protections (50) plaquées contre la face frontale des corps (30) ;
- retirer les films de protection en tirant simultanément sur les languettes de tirage ;
- rapprocher de nouveau les dispositifs l'un de l'autre jusqu'à ce que les corps soient plaqués l'un contre l'autre.

## Patentansprüche

1. Aseptische Kupplungsvorrichtung (10) für ein Rohr, wobei die Vorrichtung umfasst:
- einen rohrförmigen Einsatz (20), der einen Kanal (21) begrenzt und Folgendes aufweist: einen ersten Endabschnitt (20.1), der mit Mitteln zu seiner Verbindung mit dem Rohr versehen ist, und einen zweiten Endabschnitt (20.2), der dazu bestimmt ist, sich aus dem Rohr heraus nach außen zu erstrecken, wobei der rohrförmige Einsatz mit einem Schaft (25) versehen ist, welcher sich derart axial freitragend in dem Kanal erstreckt, dass sein freier Endabschnitt (25.1) aus dem zweiten Endabschnitt des Einsatzes herausragt; und
- einen ringförmigen Körper (30) welcher Folgendes umfasst: einen ersten Endabschnitt (30.1), der auf dem zweiten Endabschnitt des Einsatzes aufmontiert ist, und einen zweiten Endabschnitt (30.2), der sich axial vorspringend von dem zweiten Endabschnitt des Einsatzes erstreckt, um gemeinsam mit diesem im Bereich um den freien Endabschnitt des Schafts herum eine Aufnahme zur Unterbringung eines ringförmigen Dichtungselements (40) der Vorrichtung zu definieren, welches zwischen einer Stirnfläche des zweiten Endabschnitts des Einsatzes und einer einwärtsgewandten Stufe (33) des zweiten Abschnitts des ringförmigen Körpers angeordnet ist und welches zwischen einem ersten Zustand, in dem das Dichtungselement einen Durchgangsquerschnitt begrenzt, der größer als ein Querschnitt des Schafts ist, und einem zweiten Zustand, in dem das Dichtungselement den Schaft abdichtend umschließt, verformbar ist, wobei das Dichtungselement so angeordnet ist, dass es unter axialer Kompression von seinem ersten Zustand in seinen zweiten Zustand gebracht werden kann; und wobei:
- das Dichtungselement eine über den zweiten Abschnitt des ringförmigen Körpers hinaus vorstehende Stirnseite umfasst, an der eine Schutzfolie (50) lösbar befestigt ist, über welche eine Ziehlasche (51) mit einem ersten Ende, das mit einem Rand der Schutzfolie verbunden ist, und einem zweiten Ende, das sich über einen dem ersten Ende der Ziehlasche entgegengesetzten Rand der Folie hinaus erstreckt, umgeschlagen ist; und
- der erste Endabschnitt des Körpers derart auf dem Einsatz aufmontiert ist, dass er darauf axial verschiebbar ist, und zwar zwischen einer axial vorstehenden Position des Einsatzes, in welcher sich das Dichtungselement in seinem ersten Zustand befindet, und einer mit dem Einsatz flächenbündigen Position, in welcher das Dichtungselement in axialer Richtung so weit komprimiert ist, dass es seinen zweiten Zustand einnimmt.

2. Vorrichtung nach Anspruch 1, umfassend Verriegelungsmittel (28.1, 28.2, 28.3, 31, 32) des Körpers (30) in vorstehender Position und in flächenbündiger Position.

3. Vorrichtung nach Anspruch 1, umfassend Mittel (36, 37.1, 37.2) zur Vorkupplung und zur Kupplung mit einer anderen Kupplungsvorrichtung.

4. Vorrichtung nach Anspruch 3, wobei die Vorkupplungs- und Kupplungsmittel zumindest eine Verriegelungsnase umfassen, die sich über eine Stirnseite des Körpers (30) hinaus erstreckt und an einem freien Ende zumindest zwei Unebenheiten (36.1, 36.2) umfasst, die dazu bestimmt sind, mit einem Rand der anderen Kupplungsvorrichtung zusammenzuwirken.

5. Kupplungsanordnung (1) für zwei Rohre (100, 200), umfassend zwei Kupplungsvorrichtungen (10A, 10B) nach einem der vorhergehenden Ansprüche.

6. Anordnung (1) nach Anspruch 5, wobei die zwei Kupplungsvorrichtungen (10A, 10B) identisch sind.

7. Verfahren zum Kuppeln zweier Rohre (100, 200) mittels einer Kupplungsanordnung nach den Ansprüchen 5 oder 6, wobei das Verfahren die Schritte umfasst, dass:
- die Vorrichtungen koaxial einander zugewandt angeordnet werden;
- die Vorrichtungen aufeinander zubewegt werden, bis die Ziehlaschen (51) miteinander in Kontakt stehen und die Schutzfolien (50) gegen die jeweilige Stirnfläche der Körper (30) gedrückt werden;
- die Schutzfolien durch gleichzeitiges Ziehen an den Ziehlaschen abgezogen werden;
- die Vorrichtungen erneut aufeinander zubewegt werden, bis die Körper aneinandergepresst sind.

## Claims

1. An aseptic coupling device (10) for a tube, the device comprising:
· a tubular insert (20) defining a channel (21) and having both a first end segment (20.1) provided with means for connecting it to the tube and also a second end segment (20.2) for extending outside the tube, the tubular insert being provided with a cantilevered rod (25) extending axially inside the channel so as to have a free end portion (25.1) projecting out from the second end segment of the insert; and
· an annular body (30) comprising both a first end segment (30.1) mounted on the second end segment of the insert and also a second end segment (30.2) projecting axially from the second end segment of the insert so as to co-operate therewith to define a housing in register with the free end portion of the rod for receiving an annular sealing element (40) of the device that is placed between a front face of the second end segment of the insert and an internal lip (33) of the second segment of the annular body and that is deformable between a first state in which the sealing element defines a fluid flow section greater than the cross section of the rod and a second state in which the sealing element tightly surrounds the rod, the sealing element being arranged to be taken from its first state to its second state under the effect of axial compression;
and wherein:
· the sealing element has a front face projecting from the second segment of the annular body with a protective film (50) removably fastened thereto, the film having a pull-tongue (51) folded down thereon with a first end connected to an edge of the protective film and a second end projecting from the edge of the film that is opposite from the first end of the pull-tongue; and
· the first end segment of the body is mounted on the insert so as to be movable axially between a projecting position in which it projects axially from the insert and in which the sealing element is in its first state, and a flush position in which it is flush with the insert and in which the sealing element is compressed axially sufficiently to be in its second state.

2. A device according to claim 1, including locking means (28.1, 28.2, 28.3, 31, 32) for locking the body (30) in its projecting position and in its flush position.

3. A device according to claim 1, including pre-coupling and coupling means (36, 37.1, 37.2) for engaging another coupling device.

4. A device according to claim 3, wherein the pre-coupling and coupling means comprise at least one locking tab projecting axially from a front face of the body (30) and including at least two serrations (36.1, 36.2) at a free end for co-operating with an edge of the other coupling device.

5. A coupling assembly (1) for coupling together two tubes (100, 200), the coupling assembly comprising two coupling devices (10A, 10B) according to any preceding claim.

6. An assembly (1) according to claim 5, wherein the two coupling devices (10A, 10B) are identical.

7. A method of connecting together two tubes (100, 200) by means of a coupling assembly according to claim 5 or claim 6, the method comprising the following steps:
· placing the devices to face each other axially;
· moving the devices towards each other until the pull-tongues (51) are in contact with each other and the protective films (50) are pressed against the front faces of the bodies (30);
· removing the protective films by pulling simultaneously on the pull-tongues; and
· moving the devices towards each other again until the bodies are pressed against each other.
